# EUROPÄISCHE PATENTSCHRIFT

(11) **EP 0 606 269 B1**
(45) Veröffentlichungstag und Bekanntmachung des Hinweises auf die Patenterteilung: **15.01.1997**
(21) Anmeldenummer: 92919562.6
(22) Anmeldetag: 17.09.1992
(51) Int. Cl.: G01N 27/14, G01N 33/00

(54) **ABGAS-SENSOR IN PLANARTECHNOLOGIE ZUR REGELUNG VON KFZ-MOTOREN**
EXHAUST GAS SENSOR USING PLANAR TECHNOLOGY TO REGULATE MOTOR VEHICLE ENGINES
DETECTEUR DE GAZ D'ECHAPPEMENT EN TECHNOLOGIE PLANAIRE POUR LE REGLAGE DE MOTEURS DE VEHICULES AUTOMOBILES

(30) Priorität: 30.09.1991 EP 91116728
(43) Veröffentlichungstag der Anmeldung: 20.07.1994
(73) Patentinhaber: SIEMENS AKTIENGESELLSCHAFT, 80333 München (DE)
(72) Erfinder: HANRIEDER, Wolfgang, D-8000 München 70 (DE); LAMPE, Uwe, D-8058 Erding (DE); MEIXNER, Hans, D-8013 Haar (DE)
(86) Internationale Anmeldenummer: EP9202150
(87) Internationale Veröffentlichungsnummer: WO9307477

(56) Entgegenhaltungen:
- EP-A- 0 440 906
- DE-A- 2 714 040
- DE-A- 3 127 431
- DE-A- 3 808 305
- DE-A- 3 913 621
- GB-A- 1 447 488
- GB-A- 2 170 913

## Beschreibung

Die vorliegende Erfindung betrifft einen Abgas-Sensor zur Regelung von Brennkraftmaschinen, insbesondere von Kfz-Motoren, über die Bestimmung der reduzierenden Gase.

Zur Regelung von Kfz-Motoren ist außer der Kenntnis über den Sauerstoffgehalt auch die Kenntnis über den Gehalt an reduzierenden Gasen, wie CO, H₂, CH_{x,} im Abgas der einzelnen Zylinder einer Mehrzylinder-Brennkraftmaschine von Bedeutung. Mit Hilfe der betreffenden Werte können bei allen Brennkraftmaschinen-Typen Aussagen über die Vollständigkeit der Verbrennung getroffen werden. Die Ansprechzeit von dafür erforderlichen zylinderselektiven Abgas-Sensoren muß bei einer Mehrzylinder-Brennkraftmaschine im Bereich von einigen Millisekunden liegen. Die mittlere Arbeitstemperatur eines derartigen Sensors kann zwischen 300° C und 1000° C betragen.

Reduzierende Gase werden mit auf dem Markt erhältlichen Sensoren hauptsächlich nach dem Prinzip der Wärmetönnung (Benutzung von Pelistoren) oder der elektrischen Leitfähigkeit gemessen.

Ein Pelistor besteht im wesentlichen aus zwei Keramikperlen, die mit je einer Platinwendelanordnung zur Heizung und Temperaturmessung durchzogen sind. Die Platinwiderstände sind jeweils als Halbbrücke geschaltet. Eine der Keramikperlen ist mit einem Katalysatormaterial überzogen. Die bei der Reaktion der reduzierenden Gase freiwerdende Verbrennungswärme wird als Temperaturerhöhung gegenüber der zweiten Keramikperle ohne Katalysator gemessen.

Bei den Leitfähigkeits-Sensoren wird die Änderung der elektrischen Leitfähigkeit eines mit einem Katalysator versehenen Metalloxids durch die Reaktion mit reduzierenden Gasen ausgenutzt.

Aus der DE 39 13 621 A1 ist ein Gassensor für Abgas, geeignet für eine Motorsteuerung, insbesondere für die Regelung eines Magermotors bekannt. Mittels einer Kombination aus einem katalytisch wirksamen Wärmetönungsmesser und einem davon wärmeisolierten, im wesentlichen gleichartigen Bauelement ohne katalytische Wirkung wird die Vollständigkeit einer Sauerstoff/Brennstoff-Gasreaktion bestimmt.

In der GB 1 447 488 wird ein tragbarer Gasdetektor bestehend aus einem thermischen Leitfähigkeitssensor und einem katalytisch aktiven Oxidationssensor beschrieben. Jedem Sensor ist ein Referenzelement zugeordnet, so daß eine Meßbrücke entsteht.

Ein Gassensor, bestehend aus einer Heizstruktur zur Einstellung der Arbeitstemperatur und einer Mehrzahl von Sensorelementen, beispielsweise in Form von Pellistoren, wird in der GB 2 170 913 A beschrieben. Der Gassensor dient der Bestimmung der einzelnen Abgaskomponenten.

Beide Sensor-Arten werden typischerweise in Gaswarngeräten eingesetzt. In Kfz-Motoren finden hauptsächlich bislang nur λ-Sonden zur Regelung des Gemisches für die optimale Funktion des Abgaskatalysators Anwendung. Diese Sensoren sind für eine zylinderselektive Messung ebenfalls zu träge und im übrigen von ihrem physikalischen Meßprinzip her für den genannten Anwendungsbereich ungeeignet.

Der vorliegenden Erfindung liegt die Aufgabe zugrunde, einen Abgas-Sensor der eingangs genannten Art zu schaffen, der eine ausreichend kurze Reaktionszeit aufweist, in dem genannten weiten Arbeitstemperaturbereich zuverlässig arbeitet, einen kompakten Aufbau hat und kostengünstig herzustellen ist.

Zur Lösung der Aufgabe wird ein Abgas-Sensor gemäß dem Oberbegriff des Patentanspruchs 1 vorgeschlagen, der durch die darin angegebenen Merkmale gekennzeichnet ist.

Vorteilhafte Weiterbildungen der Erfindung sind durch die in den Unteransprüchen angegebenen Merkmale gekennzeichnet.

Die Wärmetönung kann dabei über die Temperaturabhängigkeit der elektrischen Leitfähigkeit von halbleitenden Metalloxiden ermittelt werden. An dem gleichen Metalloxid wird auch die reaktionsabhangige elektrische Leitfähigkeit gemessen.

Im folgenden wird die Erfindung anhand einer Figur, die ein bevorzugtes Ausführungsbeispiel betrifft, im einzelnen beschrieben.

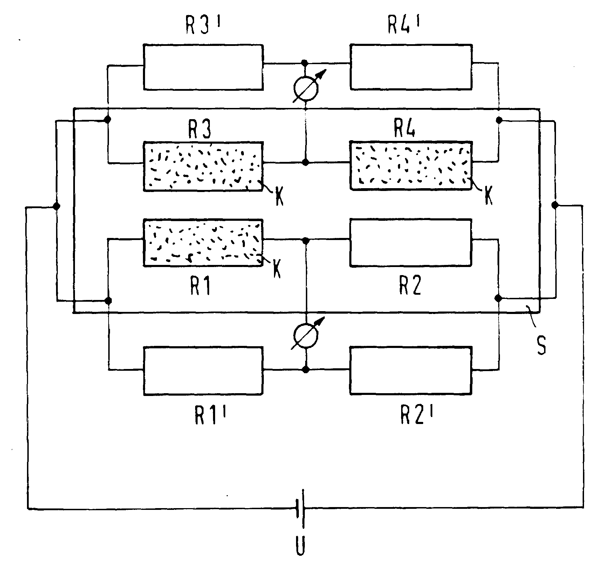

Die Figur zeigt eine schematische Darstellung einer auf einem vorzugsweise gemeinsamen Substrat S angeordneten Brückenschaltungsanordnung R1, R1', R2, R2', R3, R3', R4, R4' eines erfindungsgemäßen Abgas-Sensors.

Wie die Figur zeigt, sind von vier Metalloxid-Meßwiderständen R1, R2, R3, R4 jeweils zwei als Halb-Meßbrücke geschaltet. Vorzugsweise sind die Widerstandswerte der Widerstände der zwei Halb-Meßbrücken gleich. Jede der zwei Halb-Meßbrücken R1/R2, R3/R4 ist durch betreffende Widerstände Rl', R2' bzw. R3, R3' zu vollständigen Meßbrücken ergänzt, die nicht dem zu erfassenden Abgasstrom ausgesetzt sind. Eine der halb-Meßbrücken ist vollständig mit einer passivierenden Schicht überzogen. Bei der zweiten Halb-Meßbrücke ist nur ein Meßwiderstand mit einer passivierenden Schicht überzogen. Bei der ersten Halb-Meßbrücke ist ein Meßwiderstand, nämlich R1, bei der zweiten Halbmeßbrücke sind beide Meßwiderstände, nämlich R3, R4, mit einem Katalysatormaterial K, z. B. Pt, bedeckt. Die erste Meßbrücke aus den Widerständen R1, R2, R1', R2' dient zur Messung der Gase nach dem Wärmetönungsprinzip, die zweite Meßbrücke 2 zur Messung nach dem Leitfähigkeitsprinzip. Die gesamte Meßbruckenanordnung wird durch eine Betriebsspannung U versorgt.

Durch die Anordnung der Halb-Meßbrücken werden unerwünschte Temperaturschwankungen des Abgases sowie Langzeitdriften kompensiert.

Zur Einstellung einer mittleren konstanten Arbeitstemperatur kann der Sensor mit zusätzlichen Elementen zur Temperaturregelung versehen sein.

Um die erforderliche hohe Ansprechgeschwindigkeit zu erreichen, ist erfindungsgemäß vorgesehen, den Aufbau des Sensors in Planartechnologie (Dünnfilm- oder Dickfilmschicht) zu realisieren.

Als Substrat ist vorzugsweise ein Material vorgesehen, dessen Wärmeleitfähigkeit so groß ist, daß die Zeitkonstante für den Aufbau von Temperaturgradienten über dem Sensor in der Größenordnung der Zeiten für die Änderung der Abgaszusammensetzung liegt, beispielweise AlN.

Zum Aufbau der Elemente für die Temperaturregelung des Sensors und für die Kontaktierung und Zusammenschaltung der Metalloxidwiderstände wird vorzugsweise Platin verwendet. Für die Metalloxidwiderstände ist ein Oxid mit hoher katalytischer Aktivität und hohem Temperaturkoeffizienten der elektrischen Leitfähigkeit vorgesehen. Die Passivierung erfolgt mit einem Material mit hohem elektrischen Widerstand und guter Wärmeleitfähigkeit (z. B. Siliziumnitrid). Als Katalysator kommt ein Element oder eine Legierung der Platingruppe in Frage. Alle verwendeten Materialien müssen eine hinreichende thermische und chemische Stabilität besitzen.

Der erfindungsgemäße Sensor arbeitet bei einer konstanten mittleren Arbeitstemperatur. Zur Auswertung können die Halb-Meßbrückensignale selbst sowie deren erste und zweite zeitliche Ableitung, d. h. der stationäre Gleichgewichtswert und dessen Einstellgeschwindigkeit, herangezogen werden. Weitere Aussagen ergeben sich durch Verrechnung der Meßwerte der beiden Halb-Meßbrücken gegeneinander, z. B. durch Differenzenbildung, Summenbildung usw., und dem Vergleich mit abgespeicherten Sensor-Kennlinien.

Die Herstellung des erfindungsgemäßen Abgas-Sensors kann wie folgt ausgeführt werden:

Auf einem elektrisch isolierenden Substrat mit geeigneter Wärmeleitung werden eine Heiz- und Temperaturmeßwendelanordnung, vier Interdigitalstrukturen zur Kontaktierung der Metalloxidwiderstände, Verbindungsleitungen und Trimmwiderstände, abgeglichen durch das sog. Lasertrimmen, aus Platin aufgebracht und durch geeignete Verfahren stabilisiert. Auf die Interdigitalstrukturen wird das Metalloxid aufgetragen. Durch Lasertrimmen werden die Metalloxidwiderstände für die Arbeitstemperaturen abgeglichen. Der Sensorchip wird bis auf einen Metalloxidwiderstand mit einer Siliziumnitridschicht passiviert, und anschließend wird das Katalysatormaterial aufgebracht. Als Ergebnis staht ein Abgas-Sensor zur Verfügung, der schnell und genau den Gehalt der reduzierenden Bestandteile im Kfz-Abgas messen kann.

## Patentansprüche

1. Abgas-Sensor zur Regelung von Brennkraftmaschinen, insbesondere von Kfz-Motoren, über die Bestimmung der reduzierenden Gase,
dadurch **gekennzeichnet,**
daß auf dem gemeinsamen Substrat (S) zumindest vier Metalloxid-Meßwiderstände (R1, R2, R3, R4) angeordnet sind, von denen jeweils zwei als Halb-Meßbrücke geschaltet sind, wobei die Widerstandswerte der Widerstände der zwei Halb-Meßbrücken vorzugsweise gleich sind,
daß jede der zwei Halb-Meßbrücken (R1/R2, R3/R4) durch betreffende Widerstände (R1', R2' bzw. R3', R4'), die nicht dem zu messenden Abgasstrom ausgesetzt sind, zu vollständigen Meßbrücken ergänzt ist,
daß eine der Halb-Meßbrücken vollständig mit einer passivierenden Schicht überzogen ist und bei der zweiten Halb-Meßbrücke nur ein Meßwiderstand mit einer passivierenden Schicht überzogen ist,
daß bei der einen Halb-Meßbrücke ein einziger Meßwiderstand (R1), bei der anderen Halbmeßbrücke zwei Meßwiderstände (R3, R4) mit einem Katalysatormaterial K, vorzugsweise Pt, bedeckt ist/sind,
daß die eine Meßbrücke (R1, R2, R1', R2') mit dem einzigen mit einem Katalysatormaterial bedeckten Widerstand (Rl) zur Messung der Gase nach dem Wärmetönungsprinzip und die andere Meßbrücke (R3, R4, R3', R4') zur Messung nach dem Leitfähigkeitsprinzip dient und
daß die gesamte Meßbrückenanordnung durch eine gemeinsame Betriebsspannung (U) versorgt wird.

2. Abgas-Sensor nach Anspruch 1,
dadurch **gekennzeichnet,**
daß zur Einstellung einer mittleren konstanten Arbeitstemperatur des Abgas-Sensors zusätzliche Elemente zur Heizung und Temperaturregelung, die zumindest zum Teil auf dem gemeinsamen Substrat (S) angeordnet sind, vorgesehen sind.

3. Abgas-Sensor nach einem der Ansprüche 1 oder 2,
dadurch **gekennzeichnet,**
daß der Abgas-Sensor in Planartechnologie als Dünnfilmstruktur ausgeführt ist.

4. Abgas-Sensor nach einem der Ansprüche 1 oder 2,
dadurch **gekennzeichnet,**
daß der Abgas-Sensor in Planartechnologie als Dünnschichtstruktur ausgeführt ist.

5. Abgas-Sensor nach einem der vorhergehenden Ansprüche,
dadurch **gekennzeichnet**,
daß als Substrat (S) ein Material, beispielsweise AlN, verwendet ist, dessen Wärmeleitfähigkeit so groß ist, daß die Zeitkonstante für den Aufbau von Temperaturgradienten über dem Abgas-Sensor in der Größenordnung der Zeiten für die Änderung der Abgaszusammensetzung liegt.

6. Abgas-Sensor nach einem der Ansprüche 1 bis 4,
dadurch **gekennzeichnet,**
daß zum Aufbau der Elemente der Temperatureinstellung des Abgas-Sensors und zur Zusammenschaltung der Metalloxidwiderstände als Material Pt verwendet ist.

7. Abgas-Sensor nach einem der Ansprüche 1 bis 4,
dadurch **gekennzeichnet,**
daß für die Metalloxidwiderstände ein Oxid mit hoher katalytischer Aktivität und hohem Temperaturkoeffizienten der elektrischen Leitfähigkeit verwendet ist.

8. Abgas-Sensor nach einem der Ansprüche 1 bis 4,
dadurch **gekennzeichnet,**
daß die Passivierung mit einem Material, beispielsweise Siliziumnitrid, mit hohem elektrischen Widerstand und hoher Wärmeleitfähigkeit ausgeführt ist.

9. Abgas-Sensor nach einem der Ansprüche 1 bis 4,
dadurch **gekennzeichnet,**
daß als Katalysatormaterial ein Element oder eine Legierung der Platingruppe verwendet ist.

## Claims

1. Exhaust gas sensor for regulating internal combustion engines, in particular motor vehicle engines, by determining the reducing gases, characterized in that at least four metal oxide measuring resistors (R1, R2, R3, R4), of which in each case two are connected as a half measuring bridge, are arranged on the common substrate (S), the resistance values of the resistors of the two half measuring bridges being preferably identical, in that each of the two half measuring bridges (R1/R2, R3/R4) is supplemented by respective resistors (R1', R2' and R3', R4') which are not subject to the exhaust gas stream to be measured, to form complete measuring bridges, in that one of the half measuring bridges is completely coated with a passivizing layer and, in the case of the second half measuring bridge, only one measuring resistor is coated with a passivizing layer, in that, in the case of one of the half measuring bridges, a single measuring resistor (R1) is coated with a catalyst material K, preferably Pt, and in the case of the other half measuring bridge two measuring resistors (R3, R4) are covered with a catalyst material, K, preferably Pt, in that one of the measuring bridges (Rl, R2, R1', R2') with the single resistor (R1) which is coated with a catalyst material serves to measure the gases according to the heat tone principle and the other measuring bridge (R3, R4, R3', R4') serves to measure them according to the conductance principle, and in that the entire measuring bridge arrangement is supplied with a common operating voltage (U).

2. Exhaust gas sensor according to Claim 1, characterized in that additional elements for heating and temperature regulation, which are arranged at least partially on the common substrate (S) are provided for adjusting an average constant operating temperature of the exhaust gas sensor.

3. Exhaust gas sensor according to one of Claims 1 and 2, characterized in that the exhaust gas sensor is realized as a thin-film structure using planar technology.

4. Exhaust gas sensor according to one of Claims 1 and 2, characterized in that the exhaust gas sensor is realized as a thin-layer structure using planar technology.

5. Exhaust gas sensor according to one of the preceding claims, characterized in that the material used as a substrate (S), for example AlN, has a heat conductance which is so large that the time constant for the build-up of temperature gradients via the exhaust gas sensor is in the order of magnitude of the times for changing the composition of the exhaust gas.

6. Exhaust gas sensor according to one of Claims 1 to 4, characterized in that the material used for building up the elements of the temperature adjustment of the exhaust gas sensor and for connecting together the metal oxide resistors is Pt.

7. Exhaust gas sensor according to one of Claims 1 to 4, characterized in that an oxide is used for the metal oxide resistors which has a high catalytic activity and high temperature coefficient of the electrical conductance.

8. Exhaust gas sensor according to one of Claims 1 to 4, characterized in that the passivization with a material, for example silicon nitride, is realized with a high electrical resistance and a high heat conductance.

9. Exhaust gas sensor according to one of Claims 1 to 4, characterized in that the catalyst material used is an element or an alloy of the platinum group.

## Revendications

1. Détecteur de gaz d'échappement pour le réglage de moteurs à combustion interne, et plus particulièrement de moteurs de véhicules automobiles, par le biais de la détermination des gaz réducteurs,
**caractérisé** en ce que
au moins quatre résistances de mesure en oxyde métallique au moins (R1, R2, R3 et R4) sont disposées sur le substrat commun (S) et couplées deux par deux pour former un demi-pont de mesure, les valeurs de résistance des résistances des deux demi-ponts étant de préférence égales.
en ce que chacun des deux demi-ponts de mesure (R1/R2 et R3/R4) est complété afin de former des ponts de mesure entiers par les résistances correspondantes (R1', R2' resp. R3', R4'), ces dernières n'étant pas exposées au courant de gaz d'échappement à mesurer,
en ce que l'un des demi-ponts de mesure est recouvert dans son entièreté d'une couche destinée à le rendre passif et une seule résistance de mesure de l'autre demi-pont est recouverte d'une couche destinée à le rendre passif,
en ce qu'une seule résistance de mesure (R1) d'un des demi-ponts de mesure et deux résistances de mesure (R3 et R4) de l'autre des demi-ponts de mesure sont enduites d'un matériau catalyseur K, de préférence de Pt,
en ce que le pont de mesure (R1, R2, R1', R2') dont une seule résistance de mesure (R1) est recouverte de matériau catalyseur, sert à mesurer les gaz selon le principe de la chaleur de réaction, et l'autre pont de mesure (R3, R4, R3', R4') sert à mesurer selon le principe de la conductibilité, et
en ce que toute la disposition des ponts de mesure est alimentée par une tension de service commune (U).

2. Détecteur de gaz d'échappement selon la revendication 1,
**caractérisé** en ce que,
en matière de réglage d'une température de travail moyenne constante du détecteur de gaz d'échappement, des éléments supplémentaires sont prévus pour le chauffage et le réglage de la température, ces derniers étant disposés, au moins en partie, sur le substrat commun (S).

3. Détecteur de gaz d'échappement selon la revendication 1 ou 2,
**caractérisé** en ce que,
le détecteur de gaz d'échappement est conçu en technologie planaire sous la forme d'une structure à film mince.

4. Détecteur de gaz d'échappement selon la revendication 1 ou 2,
**caractérisé** en ce que,
le détecteur de gaz d'échappement est conçu en technologie planaire sous la forme d'une structure à couche mince.

5. Détecteur de gaz d'échappement selon l'une des revendications précédentes,
**caractérisé** en ce que,
comme substrat (S), on utilise un matériau tel que l'AlN dont la conductibilité thermique est suffisamment grande pour que la constante de temps pour la constitution de gradients thermiques à travers le détecteur de gaz d'échappement se situe dans l'ordre de grandeur des temps nécessaires à la modification de la constitution des gaz d'échappement.

6. Détecteur de gaz d'échappement selon l'une des revendications 1 à 4,
**caractérisé** en ce que,
le matériau Pt est utilisé pour la construction des éléments nécessaires au réglage de la température du détecteur de gaz d'échappement et l'interconnexion des résistances en oxyde métallique.

7. Détecteur de gaz d'échappement selon l'une des revendications 1 à 4,
**caractérisé** en ce que,
pour les résistances en oxyde métallique, on utilise un oxyde à activité catalytique élevée et à haut coefficient de température pour la conductibilité électrique.

8. Détecteur de gaz d'échappement selon l'une des revendications 1 à 4,
**caractérisé** en ce que,
la passivation est réalisée à l'aide d'un matériau tel que le nitrure de silicium, à forte résistance électrique et à conductibilité thermique élevée.

9. Détecteur de gaz d'échappement selon l'une des revendications 1 à 4,
**caractérisé** en ce que,
un élément ou un alliage appartenant à la famille du platine est utilisé comme matériau catalyseur.
